Europäisches Patentamt

European Patent Office

Office européen des brevets

(11) Veröffentlichungsnummer: **0 466 972 A1**

# EUROPÄISCHE PATENTANMELDUNG

(21) Anmeldenummer: **90122448.5**

(22) Anmeldetag: **24.11.90**

(51) Int. Cl.5: **C07D 223/22**

Ein Antrag gemäss Regel 88 EPÜ auf Berichtigung eines Schreibfehlers liegt vor. Über diesen Antrag wird im Laufe des Verfahrens vor der Prüfungsabteilung eine Entscheidung getroffen werden (Richtlinien für die Prüfung im EPA, A-V, 2.2).

(30) Priorität: **18.07.90 DE 4023204**

(43) Veröffentlichungstag der Anmeldung:
**22.01.92 Patentblatt 92/04**

(84) Benannte Vertragsstaaten:
**AT CH DE FR GB IT LI**

(71) Anmelder: **ARZNEIMITTELWERK DRESDEN GMBH**
**Wilhelm-Pieck-Strasse 35**
**O-8122 Radebeul(DE)**

(72) Erfinder: **Palitzsch, Peter, Dipl.-Chem.**
**Döbelner Strasse 114**
**O-8023 Dresden(DE)**

Erfinder: **Czernotzky, Klaus, Dipl.-Chem.**
**Scheringer Strasse 25 a**
**O-8122 Radebeul(DE)**
Erfinder: **Richter, Ehrhard, Dr.**
**Soermus Strasse 8**
**O-8122 Radebeul(DE)**
Erfinder: **Kreher, Bernd, Dr.**
**Rudolf-Harbig-Strasse 2**
**O-8122 Radebeul(DE)**
Erfinder: **Klump, Wilfried, Dipl.-Ing.**
**Gartenstrasse 35**
**O-4302 Bad Suderode(DE)**
Erfinder: **Müller,Rainer**
**Holbeinstrasse 141**
**O-8019 Dresden(DE)**

(74) Vertreter: **Puchberger, Georg, Dipl.-Ing. et al**
**Patentanwälte Dipl.-Ing. Georg Puchberger**
**Dipl.-Ing. Rolf Puchberger Dipl.-Ing. Peter**
**Puchberger Singerstrasse 13 Postfach 55**
**A-1010 Wien(AT)**

(54) **Verfahren zur Herstellung von 5-Chlorcarbonyl-5H-dibenz-/b,f/-azepin.**

(57) Die Erfindung bezieht sich auf ein Verfahren zur Herstellung von 5-Chlorcarbonyl-5H-dibenz/b,f/azepin, das die Vorstufe für die großtechnische Synthese von 5-Carbamoyl-5H-dibenz/b,f/azepin darstellt. Letztere Substanz wird als Arzneimittel verwendet.

Die wesentlichen Merkmale der Erfindung bestehen darin, daß man eine Suspension von Iminostilben in einem inerten Lösungsmittel bei 20-60 °C Phosgen einleitet, bis das Iminostilben zu einem nahezu äquimolekularen Gemisch aus 5-Chlorcarbonyl-5H-dibenz/b,f/azepin und Iminostilben-hydrochlorid umgesetzt ist, worauf man durch Zugabe einer wäßrigen Base aus dem gebildeten Iminostilbenhydrochlorid Iminostilben freisetzt und letzteres so bei Aufrechterhaltung eines sauren Reaktionsmilieus der vollständigen Phosgenierung zugänglich macht.

Nach dem vollständigen Umsatz des Iminostilbens wird die Reaktionsmischung langsam auf 80-90 °C angeheizt und unter Rühren durch die gebildete Salzsäure entgiftet.

EP 0 466 972 A1

Anwendungsgebiet der Erfindung

Die Erfindung betrifft ein technisches Verfahren zur Herstellung von 5-Chlorcarbonyl-5H-dibenz/b,f/azepin (II). Diese Verbindung stellt die Vorstufe für die großtechnische Synthese von 5-Carbamoyl-5H-dibenz/b,f/azepin (III) dar, das als Arzneimittel, vorzugsweise als Antiepileptikum, verwendet wird.

Charakteristik der bekannten technischen Lösungen

Die Synthese des 5-Chlorcarbonyl-5H-dibenz/b,f/azepins (II) aus Iminostilben (I) (= 5H-Dibenz/b,f/azepin) wurde erstmalig von W.Schindler beschrieben (DE-AS 1 136 707, CH-PS 54 023). Nach diesem Verfahren wird das Iminostilben (I) in Toluen suspendiert und in diese Suspension wird Phosgen eingeleitet, wobei sich die Reaktionsmischung bis auf 70°C erwärmt. Anschließend wird die Reaktionsmischung unter weiterem Einleiten von Phosgen zum Rückfluß angeheizt und so lange gekocht, bis das Iminostilben vollständig umgesetzt und die Chlorwasserstoffentwicklung beendet ist.
Sobald die Reaktionslösung frei von Iminostilben ist, wird die Phosgeneinleitung abgestellt. Das überschüssige Phosgen wird aus der Reaktionsmischung mit trockenem Stickstoff oder trockener Luft entfernt (s. hiezu DE-AS 1 001 271, wo das überschüssige Phosgen nach der beendeten Phosgenierung des 5H-10,11-Dihydro-dibenz/b,f/azepins = Iminodibenzyl gleichfalls mit trockener Luft ausgeblasen wird). Die derartig entgiftete Reaktionslösung wird, wie üblich, aufgearbeitet und das durch Kristallisation isolierte 5-Chlorcarbonyl-5H-debenz/b,f/azepin (II) wird in bekannter Weise zum 5-Carbamoyl-5H-dibenz/b,f/azepin (III) amidiert. Die bis zum heutigen Zeitpunkt beschriebenen Verfahren arbeiten alle in inerten,wasserfreien Lösungsmitteln bei Temperaturen über 100°C (beispielsweise in Toluen, in Chlorbenzen oder in o-Dichlorbenzen; siehe hiezu in Betracht gezogene Patentschriften und Übersichtsartikel von B.Renfroe, C.Harrington und G.R. Proctor in "Heterocyclic compounds", Vol.43, "Azepines", Teil I, John Wiley & Sons, New York 1984, Seite 524, Tabelle 118).
Die Dissoziation des bei der Phosgenierung stets entstehenden Iminostilben-Hydrochlorids in Chlorwasserstoffgas und freies Iminostilben wird bei allen technischen Verfahren thermisch vorgenommen, indem man das Reaktionsgemisch bis zum Siedepunkt des indifferenten Lösungsmittels anheizt und unter Rückfluß Phosgen einleitet. Die nach dem Stand der Technik durchgeführten Heißtemperaturphosgenierungen arbeiten alle bei 100°C und höher, um eine vollständige Phosgenierung des Iminostilbens oder Iminodibenzyls zu erreichen.
Die bekannten Verfahren zur Herstellung von Carbamidsäurechloriden aus sekundären Aminen sind im Houben-Weyl (Band E 4, Seiten 46-50, 1983) in einer Tabelle zusammengefaßt. Als Lösungsmittel werden in der Regel vor allem aromatische Kohlenwasserstoffe, wie Benzen, Toluen oder Chlorbenzen eingesetzt. Es sind jedoch auch Verfahren in CHCl₃, in 1,4-Dioxan und in Essigsäureethylester beschrieben worden. Arbeitet man bei niederen Temperaturen, so wird bei der Einleitung von Phosgen in eine Lösung des sekundären Amins in einem inerten Lösungsmittel nur die Hälfte des Amins in das gewünschte Carbamidsäure-chlorid überführt,weil der bei der Reaktion freiwerdende Chlorwasserstoff die andere Hälfte des Amins in das Hydrochlorid überführt. Das Amin-hydrochlorid scheidet sich in kristalliner Form ab, d.h., die Ausbeute an Darbamidsäurechlorid kann im günstigsten Fall nur 50% betragen.
Seit der Arbeit von H.Erdmann und P.Huth (J.Prakt.Chem. (2) 56, 7 (1987) ) ist bekannt, daß man den Umsatz vervollständigen kann, wenn man eine inerte wasserfreie Base, wie Pyridin, in mindestens äquimolekularer Menge zusetzt.
Nach Houben-Weyl (s.o.) sind als inerte Basen außer Pyridin noch Triethylamin und naturgemäß das umzusetzende Amin selbst geeignet. Da man stets mindestens äquimolekulare Mengen an inerter Base benötigt, verteuert sich die Kaltphosgenierung und der Verfahrensablauf wird technologisch aufwendig, denn das Amin-hydrochlorid muß auf jeden Fall zwecks Rückgewinnung der inerten Base abgetrennt werden. Aus diesem Grund besitzt die Kaltphosgenierung in Gegenwart von inerten Hilfsbasen nur Bedeutung für die Umsetzung von temperaturempfindlichen, sekundären Aminen, die bei den hohen Temperaturen der Heißphosgenierung verstärkt zu unerwünschten Nebenreaktionen neigen.
In der Technik arbeitet man bevorzugt bei Temperaturen oberhalb von 100°C. Im Houben-Weyl (s.o.) wird hiezu ausgeführt:
"Vorteilhafterweise erhitzt man das Reaktionsgemisch unter weiterem Einleiten von Phosgen auf über 100°C, wobei das Amin-hydrochlorid in das Carbamidsäurechlorid übergeht". Das bei dieser thermischen Dissoziation freiwerdende HCl-Gas reißt beträchtliche Mengen Phosgen mit. Aus diesem Grund muß das Abgas in speziellen Abgasanlagen entgiftet und vernichtet werden. Im Havariefall kann eine derartige Verfahrensweise zu einer ernsthaften Gefährdung der Umwelt führen. (Aufgrund der extremen Giftigkeit des

2

Phosgens besteht vor allem eine Gefahr für angrenzende Wohngebiete).

Die Arbeitsweise der Heißphosgenierung hat vor allem die folgenden schwerwiegenden Nachteile:

- hohe Abgasbelastungen durch freigesetztes HCl-Gas, durch mitgerissenes Phosgen und mitgeführte Lösungsmitteldämpfe und die daraus resultierenden Umweltschutzprobleme,
- lange Reaktionszeiten über 18-24 Stunden mit starken, korrosiven Belastungen der Anlagen,
- hohe energetische Aufwendungen und
- eine Vielzahl von Nebenreaktionen sowie Dunkelfärbung des Reaktionsproduktes, die letzten Endes zu entscheidenden Qualitätsminderungen des 5-Carbamoyl-5H-dibenz/b,f/azepins führen, sowie die
- oberhalb von 90 °C verstärkt stattfindende Verengung des Siebenrinringes des Iminostilbens unter Bildung von unerwünschtem 9-Methylacridin.

Das Iminostilben ist in die Gruppe der temperaturempfindlichen Amine einzuordnen. Aus diesen Gründen wird das Iminostilben vorteilhafterweise nach dem Verfahren Schindler (DE-AS 1 136 707) phosgeniert.

Die thermische Dissoziation des Iminostilben-hydrochlorids in freies Iminostilben und HCl-Gas erfolgt erst bei ca. 90 °C hinreichend schnell, um für industrielle Zwecke akzeptable Reaktionszeiten zu erreichen.

Die von Schindler bevorzugte Variante der Zweiteilung der Phosgenierung in eine Phase der Kaltphosgenierung (Umsatz der ersten Hälfte, 50%) und eine Phase der Heißphosgenierung (Umsatz der zweiten Hälfte) besitzt gegenüber der direkten Heißphosgenierung eindeutige Vorteile, denn erstens wurde auf diese Weise die Ausbeute beträchtlich erhöht, zweitens wurden die oberhalb von 90 °C ablaufenden Nebenreaktionen zurückgedrängt und drittens wurden die Qualität und die Farbe verbessert. Trotzdem bleiben aber die beschriebenen Nachteile in der zweiten Reaktionsstufe - vom Beginn des Anheizens auf 90 °C und während der thermischen Dissoziation des Iminostilben-hydrochlorids bis zum Endpunkt der Reaktion - erhalten.

Um die schonenden Reaktionsbedingungen der Kaltphosgenierungsphase möglichst vollständig auszunutzen, wird ein Phosgenüberschuß in die Reaktionsmischung eingeleitet. Heizt man anschließend die Reaktionsmischung zur thermischen Dissoziation des Iminostilben-hydrochlorids an, so kann es zum Druckstoß kommen. Auf diese Gefahr wird im Houben-Weyl (Band E 4, Seite 744, 1983) ebenfalls aufmerksam gemacht.

Bei einem Druckstoß reißen die spontan freigesetzten HCl-Gasmengen beträchtliche Mengen Phosgen mit. Die Abgasvernichtungsanlage bzw. -entgiftungsanlage muß folglich groß genug dimensioniert sein, um einen derartigen Druckstoß des Phosgens in die Atmosphäre zu vermeiden.

Um die unerwünschten Nebenreaktionen und die Bildung von 9-Methylacridin zurückzudrängen, arbeitet man zweckmäßig bei Temperaturen von 90 - 100 °C. Bei dieser Reaktionstemperatur verläuft zwar die Phosgenierung hinreichend schnell, aber der Dampfdruck des Phosgens ist gegenüber der Phase der Kaltphosgenierung naturgemäß beträchtlich erhöht, so daß es nicht zu verhindern ist, daß der freigesetzte Chlorwasserstoff ständig große Mengen Phosgen mitführt. Dies äußert sich schon in der Tatsache, daß für den Umsatz der ersten Hälfte Iminostilben in der Phase der Kaltphosgenierung wesentlich weniger Zeit benötigt wird als für den Umsatz der zweiten Hälfte in der Phase der Heißphosgenierung. Nach dem vollständigen Umsatz des Iminostilbens muß die Reaktionslösung entgiftet werden.In der Regel wird das überschüssige Phosgen aus der heißen Reaktionslösung mit trockenem Stickstoff ausgeblasen, oder es wird ein Teil des Lösungsmittels abdestilliert, bis die Reaktionsmischung phosgenfrei ist. Diese Verfahrensweisen der Entgiftung besitzen den Nachteil, daß bei Undichtigkeiten aufgrund des in der Anlage herrschenden Gasdruckes Phosgen austreten kann. Über längere Zeiträume besteht folglich die Gefahr, daß die Umwelt durch Phosgen belastet werden kann.

In der Literatur sind außer dem beschriebenen Verfahren zur direkten Phosgenierung des Iminostilbens (I) noch einige Verfahren zur Synthese des 5-Chlorcarbonyl-5H-dibenz/b,f/azepins (II) beschrieben worden, die vom Imino-dibenzyl (IV) ( = 10,11-Dihydro-5H-dibenz/b,f,azepin) ausgehen (siehe hiezu GB- PS 1 246 606, DD-PS 82 719, DD-PS 100 948, DD-PS 101 671, DD-PS 102 149, DD-PS 102 150, DD-PS 102 151, DD-PS 108 535, DD-PS 133 052, DD-PS 234 862 A1, DD-PS 234 863 A1).

Nach diesen Verfahren wird Iminodibenzyl (IV) in einem siedenden inerten aromatischen Lösungsmittel - vorzugsweise Toluen oder Chlorbenzen - mit Phosgen umgesetzt, wobei das Phosgen in den Rücklauf eingeleitet wird. Es handelt sich folglich wieder um eine Heißphosgenierung mit allen ihren Nachteilen.

Das erhaltene 5-Chlorcarbonyl-5H-10,11-dihydro-dibenz/b,f/azepin (V) wird anschließend in einem inerten organischen Lösungsmittel mit elementarem Brom oder einem selektiven Bromierungsreagenz umgesetzt, wobei die entsprechenden 10-Monobrom- (VI, R = H) und/oder 10,11-Dibrom-Derivate (VII, R = Br) gebildet werden. Anschließend werden die Bromverbindungen (VI und/oder VII) thermisch dehydrobromiert und/oder debromiert. Bei diesem thermischen Prozeß erfolgt gleichzeitig ein teilweiser Austausch (30-40%) des Chloratoms der 5-Chlorcarbonyl-Gruppe gegen ein Bromatom.

Diese drastischen Reaktionsbedingungen führen zwangsläufig aufgrund der erforderlichen hohen Reaktionstemperaturen (150 - 170°C) und des freiwerdenden Broms zu nicht kontrollierbaren Nebenreaktionen (Kernbromierungen, Verharzungen, Crackung, Verfärbung).

Daraus folgt, daß das über derartige Prozesse synthetisierte 5-Chlorcarbonyl-5H-dibenz/b,f/azepin (II) außer zahlreichen - vor allem bromhaltigen - Nebenprodukten noch schmierige, teerige Anteile und Farbstoffe enthält, deren Entfernung einen enormen Aufwand erfordert.

Über die Natur und Struktur der erwähnten Nebenprodukte liegen keine Kenntnisse vor. Die üblichen Reinigungsverfahren führen zu beträchtlichen Verlusten.

Bisher existiert kein Reinigungsverfahren, daß in ökonomisch vertretbarer Weise das Problem des Restbromgehaltes löst. Das alles ist erklärbar, denn der laut HPLC ermittelte Gehalt des auf diese Weise synthetisierten 5-Chlorcarbonyl-5H-dibenz/b,f/azepins (II) liegt bei durchschnittlich 90%, d.h. das Vorprodukt - für das Finalprodukt Carbamazepin - enthält ca. 10% Verunreinigungen.

Ziel der Erfindung

Die Erfindung verfolgt das Ziel, 5-Chlorcarbonyl-5H-dibenz/b,f/azepin der Formel (II) unter äußerst schonenden und milden Reaktionsbedingungen in quantitativer Ausbeute bei wesentlich verbesserter Qualität aus Iminostilben herzustellen, wobei das neuartige Verfahren mit wesentlich verkürzter Reaktionszeit und mit erheblich geringerem Energiebedarf auskommt. Mit der Erfindung sollen ferner eine deutliche Verbesserung des Umweltschutzes und merklich verringerte Gefährdungsmomente erzielt werden.

Darlegung des Wesens der Erfindung

Der vorliegenden Erfindung liegt die Aufgabe zugrunde, 5-Chlorcarbonyl-5H-dibenz/b,f/azepin der Formel (II) bei verkürzter Reaktionszeit unter Vermeidung der Heißphosgenierungsphase bzw. unter Ausschaltung der bisher üblichen thermischen Dissoziation des Iminostilbenhydrochlorids in quantitativer Ausbeute und in wesentlich verbesserter Qualität herzustellen. Außerdem ist durch die Reaktionsführung die Phosgenemission zu beseitigen und eine Selbstentgiftung der Reaktionslösung zu gewährleisten.

Entsprechend der vorliegenden Erfindung wird das dadurch erreicht, daß man in eine Suspension von Iminostilben (I) in einem inerten Lösungsmittel bei 20 - 60°C Phosgen einleitet oder eine Lösung von Phosgen im gleichen Lösungsmittel zulaufen läßt, bis das Iminostilben zu einem nahezu äquimolekularen Gemisch aus 5-Chlorcarbonyl-5H-dibenz/b,f/azepin (II) und Iminostilbenhydrochlorid umgesetzt ist, worauf man durch Zugabe einer wäßrigen Base aus dem gebildeten Iminostilbenhydrochlorid Iminostilben freisetzt und so bei Aufrechterhaltung eines sauren Reaktionsmilieus der vollständigen Phosgenierung zugänglich macht.

Vorzugsweise wird im Temperaturbereich von 35-50°C gearbeitet. Nach dem etwa 50%igem Umsatz des Iminostilbens dem gewünschten Carbamidsäurechlorid (II) beginnt man unter ständigem weiteren Einleiten von Phosgen entweder mit dem Zulauf von einer Lösung eines Alkalihydroxides oder von verdünntem Ammoniakwasser oder einer wäßrigen Lösung eines Alkalicarbonates bzw. -hydrogencarbonates oder einer wäßrigen Lösung eines infolge Hydrolyse alkalisch reagierenden Salzes (beispielsweise Natriumacetat).

Während des Zulaufes der wäßrigen Base wird die Temperatur bei 35-50°C gehalten.

Nach dem Zulauf der wäßrigen Base wird die Einleitung des Phosgens bis zum 100%igen Umsatz des Iminostilbens fortgesetzt, wobei die angegebenen Temperaturgrenzen eingehalten werden. Der vollständige Umsatz des Iminostilbens wird dünnschichtchromatografisch ermittelt.

In der Endphase der Phosgenierung hält man die Temperatur etwa bei 40-50°C, damit das gebildete 5-Chlorcarbonyl-5H-dibenz/b,f/azepin (II) nicht auskristallisiert. Temperaturabweichungen um einige Grade nach oben oder unten besitzen keine Bedeutung.

Sobald in der Reaktionslösung kein Iminostilben mehr nachweisbar ist, wird die Phosgeneinleitung beendet. Danach wird die stark saure Reaktionsmischung (die den pH-Wert 1 besitzt) langsam auf 80-90°C erwärmt, so daß das überschüssige Phosgen durch die in der wäßrigen Phase vorhandene Salzsäure hydrolysiert wird. Auf diese Weise wird die Reaktionsmischung in wesentlich kürzerer Zeit und einfacher entgiftet als nach dem Ausblasverfahren der unter Ausschluß von Wasser durchgeführten Heißphosgenierung.

Es ist außerordentlich überraschend, daß nach dem erfindungsgemäßen Verfahren weder das bereits gebildet 5-Chlorcarbonyl-5H-dibenz/b,f/azepin (II) noch das eingeleitete Phosgen durch die zugesetzte wäßrige Base oder die gegen Ende Phosgenierung vorliegende,salzsaure, wäßrige Phase in irgendeiner Form merklich zersetzt werden.

Phosgen wird beispielsweise mit 15-20%iger Natronlauge entgiftet (s. "Organikum", 6. Auflage, Seite 630, 1967). Das 5-Chlorcarbonyl-5H-dibenz/b,f/azepin (II) wird als alkali empfindliches Carbamidsäurechlorid ebenfalls in Gegenwart von wäßrigen Alkalilaugen sofort spontan über das Natriumsalz der betreffenden Carbamidsäure unter Decarboxylierung wieder in das Iminostilben überführt.

Das sind die Gründe, warum man Amine normalerweise nicht in Gegenwart wäßrig-alkalischer Lösungen mit Phosgen umsetzen kann. In völlig unerwarteter Weise verläuft die Phosgenierung des Iminostilbens unter den beschriebenen Bedingungen außerdem wesentlich schneller und unter deutlich milderen, schonenderen Bedingungen. Die Reaktionszeit wird nach dem erfindungsgemäßen Verfahren auf die Hälfte verkürzt. Die Bildung der Nebenprodukte wird ebenfalls zurückgedrängt. Unter den beschriebenen Reaktionsbedingungen wird erstaunlicherweise nur sehr wenig 9-Methyl-acridin gebildet. Dieses unerwünschte Nebenprodukt wird nach dem erfindungsgemäßen Verfahren auf sehr elegante Art vorteilhafterweise nach der "Selbstentgiftung" bei der Phasentrennung entfernt.

Die entgiftete, heiße Reaktionsmischung bleibt bei 80-90 °C zur Phasentrennung eine gewisse Zeit stehen. Die untere wäßrige, stark saure Phase wird abgetrennt und verworfen. In dieser wäßrigen Phase sind - außer dem jeweiligen Salz - ungefähr 0,2% der ursprünglich eingesetzten Iminostilbenmenge in Form der verschiedensten Nebenprodukte enthalten. Davon entfallen schätzungsweise 50% auf das 9-Methyl-acridin, das als Hydrochlorid vorliegt. Iminostilben ist in der sauren wäßrigen Lösung in äußerst geringer Menge vorhanden. Die Entgiftung der Reaktionslösung durch die gebildete 7-10%ige Salzsäure besitzt folglich noch einen positiven Effekt, indem basische,aminartige Nebenprodukte in Form ihrer Hydrochloride aus der organischen Phase extrahiert werden, d.h. die wäßrige, saure Phase wirkt in bezug auf das Endprodukt Carbamazepin im Sinne einer Vorreinigung.

Als inerte Lösungsmittel eignen sich sowohl die aromatischen Kohlenwasserstoffe Benzen, Toluen oder Chlorbenzen als auch die aliphatischen Chlorkohlenwasserstoffe Chloroform oder Tetrachlorkohlenstoff, wobei vorzugsweise Toluen oder Chlorbenzen verwendet werden.

Als Basen werden vorzugsweise verdünnte Alkalilaugen oder verdünntes Ammoniakwasser eingesetzt. Die Verwendung dieser Basen - als Vorzugsvariante - garantiert ein abgasfreies Phosgenierungsverfahren bis zum nahezu vollständigen Umsatz des Iminostilbens, d.h., erst kurz vor dem Ende der Reaktion beginnt eine geringfügige Zersetzung des eingeleiteten Phosgens, die sich in einer Kohlendioxid entwicklung äußert. Das entweichende Kohlendioxid trägt geringe Mengen Phosgen in die Absorptionsvorlage aus. Gegenüber der bisher praktizierten Heißphosgenierung sind das jedoch nur noch einige wenige Prozente der sonst ausgetriebenen und zu entgiftenden Phosgen- und HCl-haltigen Abgase.

Nach dem erfindungsgemäßen Verfahren wird ein äquimolekularer Teil des freigesetzten Chlorwasserstoffs durch die zugesetzte Base chemisch gebunden, während der Rest in der wäßrigen Phase gelöst wird.

Der Einsatz der wäßrigen Lösungen der Alkalicarbonate bzw. -hydrogencarbonate ist prinzipiell möglich. In diesem speziellen Fall setzt aber mit dem Beginn des Zulaufes dieser Lösungen zum Reaktionsgemisch sofort eine Kohlendioxidentwicklung ein, so daß spätestens nach dem 50%igen Umsatz Phosgen in das Abgas gelangt. Obwohl hier weniger Phosgen ausgetragen wird als nach dem Heißphosgenierungsverfahren, stellt die Verwendung von Alkalilaugen oder Ammoniakwasser die bevorzugte Verfahrensvariante dar.

Der Einsatz der letzteren Basen ist jedoch von Reaktionsbeginn an nicht möglich, denn unter alkalischen Bedingungen - d.h. in Gegenwart überschüssiger wäßriger Baselösungen - wird das Phosgen sofort zersetzt.

Schwerlösliche,nahezu neutral reagierende Carbonate, wie beispielsweise Calciumcarbonat,können als wäßrige Suspension bereits zu Beginn der Phosgenierung als Säurefänger eingesetzt werden. Aber in diesem Fall setzt die $CO_2$-Entwicklung - und damit das Austreiben von Phosgen - wesentlich zeitiger ein als beim Zulauf einer wäßrigen Alkalicarbonatlösung nach dem ca. 50%igen Umsatz des Iminostilbens.

Die wäßrigen Lösungen der Alkalicarbonate oder die wäßrigen Suspensionen der Alkalihydrogencarbonate oder Erdalkalicarbonate sind für großtechnische Phosgenierungen (Ansatzgröße bis 600 kg Iminostilben) deshalb nicht sonderlich geeignet.

Eine spezielle Ausführungsform der Erfindung besteht jedoch darin, daß man nach dem ca. 50%igen Umsatz des Iminostilbens eine wäßrige Suspension eines Erdalkalioxides - vorzugsweise CaO oder MgO - bzw. Erdalkalihydroxides zulaufen läßt.

Für ein Äquivalent Iminostilben setzt man 0,55 - 1,00 Äquivalente Base ein - vorzugsweise jedoch nur 0,55 - 0,75 Äquivalente Base.

Beispielsweise benötigt man für 1 mol Iminostilben 0,55 - 0,75 mol NaOH, KOH oder $NH_3$ bzw. 0,28 - 0,38 mol CaO.

Eine weitere besondere Ausführungsform des erfindungsgemäßen Verfahrens besteht auch darin, daß man bereits nach ca. 10-20%igem Umsatz des Iminostilbens mit dem Zulauf der wäßrigen Baselösung beginnt, wobei man die Einleitungsgeschwindigkeit des Phosgens und die Zulaufgeschwindigkeit der wäßrigen Base so regelt, daß der pH-Wert der Reaktionsmischung nie in den alkalischen Bereich abgleitet.

5

Ausführungsbeispiele

Beispiel 1

160 g Iminostilben (I) (0,835 mol) werden in 800 ml Toluen suspendiert. Diese Suspension wird auf 35-40°C erwärmt. In die gerührte Suspension leitet man Phosgen ein. Die Reaktion ist leicht exotherm. Die Temperatur der Reaktionsmischung steigt auf ungefähr 50°C an. Bei großtechnischen Ansätzen wird die Temperatur der Reaktionsmischung durch Kühlung bei 40-50°C gehalten.

Nach dem 50%igen Umsatz (ca. 1,5 Std. Reaktionszeit) des Iminostilbens zum 5-Chlorcarbonyl-5H-dibenz/b,f/azepin (II) steigt die Temperatur der Reaktionsmischung nicht mehr an und die Iminostilbenfarbe ist verschwunden. Unter ständigem weiteren Einleiten von Phosgen beginnt man an diesem Punkt mit dem langsamen Zulauf von verdünnter Natronlauge (20 g NaOH = 0,50 mol gelöst in 150 ml Wasser).

Der Zulauf der Lauge ist nach 30-45 min beendet. Das Iminostilbenhydrochlorid geht in Lösung. Es wird weiter Phosgen eingeleitet.

Die Reaktionslösung nimmt mit fortschreitender Reaktion eine immer hellere Farbe an. Gegen Ende der Reaktion geht die Farbe des Schaumes der Reaktionslösung von gelbbraun über gelb in weiß über. Diese weiße Schaumkrone auf der gerührten Reaktionslösung und die beginnende $CO_2$-Entwicklung kurz vor dem Ende der Reaktion sind wichtige Hinweise auf den vollständigen Umsatz des Iminostilbens.

Gegen Ende der Reaktion wird die Temperatur bei nahezu 50°C gehalten, damit das gebildete Chlorcarbonyl-iminostilben nicht auskristallisiert. Die Phosgeneinleitung wird beendet, wenn in der Reaktionslösung kein Iminostilben mehr nachweisbar ist. Es werden 100 ml Wasser zugesetzt, um das gebildete NaCl in Lösung zu halten.

Anschließend heizt man die Reaktionsmischung, die den pH-Wert 1 besitzt, langsam von 50°C auf 80-90°C an. Überschüssiges Phosgen wird unter diesen Bedingungen schnell hydrolytisch zersetzt.

Sobald die Reaktionsmischung phosgenfrei ist, wird die untere wäßrige, saure Phase abgetrennt und verworfen. Diese wäßrige Phase wirkt reinigend auf das gebildete 5-Chlorcarbonyl-5H-dibenz/b,f/azepin (II), denn sie enthält mehrere aminartige Nebenprodukte (0,2 - 0,3 g) in Form ihrer Hydrochloride, vor allem 9-Methylacridin. Aus der wäßrigen Phase können die aminartigen Nebenprodukte mit NaOH freigesetzt werden.

Die toluenische Phase kann mit verdünnter Salzsäure extrahiert und mit Wasser nachgewaschen werden, wenn dies erforderlich ist. Nach erneuter Phasentrennung wird die toluenische Lösung des Chlorcarbonyl-iminostilbens (II) destilliert. Nach der Entfernung des Lösungsmittels wird die verbleibende Schmelze des Chlorcarbonyl-iminostilbens in Methanol gefällt. Die methanolische Suspension wird abgekühlt und das kristallisierte Chlorcarbonyl-iminostilben wird abgesaugt und getrocknet.

```
Ausbeute:        197   - 200 g 5-Chlorcarbonyl-5H-dibenz/b,f/
                              azepin (II)

                  93   -  94 % d.Th.

F:               157   - 158°C

Gehalt lt. HPLC: 99,8 - 100%ig

Gehalt lt. DC:   99,7 -  99,9%ig
```

Das auf diese Weise erhaltene 5-Chlorcarbonyl-5H-dibenz/b,f/azepin (II) liefert nach den in der Patentliteratur beschriebenen Amidierungsverfahren ein Roh-Carbamazepin, das bereits nach einer einzigen Umkristallisation aus Methanol-Wasser ein Rein-Carbamazepin ergibt, das allen Anforderungen der internationalen Pharmakopöen entspricht (maximale Einzelverunreinigung ≦ 0,01%).

Aus der methanolischen Fällungsmutterlauge erhält man nach dem Einengen weitere 5 - 10 g 5-Chlorcarbonyl-5H-dibenz/b,f/azepin (II).

Die Gesamtausbeute beträgt somit 205 - 207 g, das sind 96,5 - 97,5% d.Th.

Das aus der methanolischen Mutterlauge gewonnene Chlorcarbonyliminostilben (II) wird gesammelt und gesondert verarbeitet.

Beispiel 2

160 g Iminostilben werden in 800 ml Toluen suspendiert. In diese Suspension wird nach Beispiel 1 bei 35-40°C Phosgen eingeleitet. Nach dem 50%igen Umsatz des Iminostilbens beginnt man mit dem langsamen Zulauf von 70 ml 12,7%igem Ammoniakwasser (Dichte = 0,95 g/cm³ bei 15°C; 0,50 mol NH₃), wobei die Phosgeneinleitung ständig fortgesetzt wird. Das verdünnte Ammoniakwasser wird in ca. 1 Std. zugetropft.

Anschließend wird die Phosgeneinleitung bis zum vollständigen Umsatz des Iminostilbens fortgesetzt. Danach wird die Reaktionslösung langsam von ca. 50°C auf 80-90°C angeheizt und bei dieser Temperatur gerührt, bis kein Phosgen mehr nachweisbar ist (Dauer der Entgiftung ca. 1 Std.). Die entgiftete Reaktionsmischung wird mit 100 ml Wasser versetzt, um das Ammoniumchlorid zu lösen.

Danach werden 4 g Aktivkohle zugegeben. Die Mischung wird eine weitere Stunde bei 80-90°C gerührt und anschließend filtriert. Die Kohle wird mit 50 ml heißem Toluen gewaschen. Vom Filtrat wird die saure wäßrige Phase abgetrennt und verworfen. Die toluenische Lösung wird nach Beispiel 1 behandelt.

```
Ausbeute:            195   - 205 g 5-Chlorcarbonyl-5H-dibenz/b,f/
                                   azepin (II)
                     92,0 -  96,5% der Theorie
F.                   157   - 158°C
Gehalt lt.HPLC:      99,8 - 100%ig
```

Aus der methanolischen Fällbadmutterlauge erhält man nach der Destillation weitere 3 - 10 g Chlorcarbonyl-iminostilben (II).

Beispiel 3

160 g Iminostilben werden in 800ml Chlorbenzen suspendiert. Diese Suspension wird nach Beispiel 1 mit Phosgen behandelt. Nach dem 50%igen Umsatz des Iminostilbens beginnt man mit dem Zulauf von verdünnter Kalilauge (30 g KOH = 0,55 mol, gelöst in 170 ml Wasser). Die Lauge wird unter weiterer Phosgeneinleitung in ungefähr 45 Minuten gleichmäßig zugetropft.

Während der Phosgenierung wird die Temperatur bei 35-45°C gehalten, vorzugsweise bei 34-40°C. Aufgrund der besseren Löslichkeit des 5-Chlorcarbonyl-5H-dibenz/b,f/azepins (II) in Chlorbenzen (gegenüber Toluen) kann bei ca. 40°C gearbeitet werden, ohne daß das Reaktionsprodukt zu kristallisieren beginnt. In Toluen muß unterhalb von 40°C mit der Kristallisation des Chlorcarbonyliminostilbens (II) gerechnet werden. Nach dem vollständigen Umsatz des Iminostilbens wird die Reaktionsmischung wie in Beispiel 1 oder 2 entgiftet. Um das gebildete KCl in Lösung zu halten, werden 80 ml Wasser zugesetzt.

Die Reaktionsmischung wird anschließend mit 4 g Aktivkohle versetzt, 1 Stunde bei 80-90°C gerührt und danach filtriert.

Die Kohle wird mit 50 ml heißem Chlorbenzen gewaschen. Die wäßrige Phase wird bei 80-90°C (evtl. unter Zusatz eines oberflächenaktiven Stoffes zur Brechung der Emulsionsschicht) abgetrennt und verworfen. Die Chlorbenzenphase wird nach Beispiel 1 aufgearbeitet.

```
Ausbeute:            190   - 193 g 5-Chlorcarbonyl-5H-dibenz/b,f/
                                   azepin (II)
                     90    -91,5 % d.Th.
F.                   157   - 158°C
Gehalt lt HPLC:      99,75 - 100%ig
```

Aus der methanolischen Fällbadmutterlauge erhält man weitere 10-15 g Chlorcarbonyl-iminostilben (II)

Beispiel 4

160 g Iminostilben werden in 800 ml Chlorbenzen suspendiert und bei 35-50°C mit Phosgen behandelt, bis die Iminostilbenfarbe verschwunden ist. Zu dem annähernd äquimolekularen Gemisch aus 5-Chlorcarbonyl-5H-dibenz/b,f/azepin (II) und Iminostilben-hydrochlorid tropft man unter weiterer Phosgeneinleitung innerhalb von 1 Std. eine Lösung von 41 g (0,50 mol) Natriumacetat in 150 ml Wasser, wobei die Temperatur der Reaktionsmischung auf 40°C absinken darf. Nach dem Zulauf der Acetatlösung wird bei 40-45°C weiter Phosgen eingeleitet bis zum vollständigen Umsatz des Iminostilbens. Anschließend wird die Reaktionslösung auf 80-90°C angeheizt und entgiftet. Die phosgenfreie Reaktionsmischung wird in einen Scheidetrichter überführt. Nach der Abtrennung der nach Essigsäure riechenden, wäßrigen Phase wird die Chlorbenzenlösung,wie in Beispiel 1 bis 3 beschrieben, aufgearbeit.

```
Ausbeute:        192   - 194 g  5-Chlorcarbonyl-5H-dibenz/b,f/
                                 azepin (II)
                 90,5 - 91,5%  d.Theorie
F:               156   - 158°C
```

Beispiel 5

160 g Iminostilben werden in 800 ml Toluen suspendiert und nach Beispiel 1 mit Phosgen umgesetzt. Nach dem 50%igen Umsatz des Iminostilbens beginnt man bei ca. 40°C unter weiterer Phosgeneinleitung mit der langsamen Zudosierung von 14 g (0,25 mol) CaO in 170 ml Wasser (diese Suspension wird gerührt, um sie homogen zu halten). Die Zugabe der CaO-Wasser-Suspension erfolgt in ca. 45 min.

Anschließend wird bis zum totalen Iminostilbenumsatz weiter Phosgen eingeleitet, wobei nach Möglichkeit bei 40 - 45°C gearbeitet wird. Die Reaktionsmischung wird, wie üblich, entgiftet und danach, wie in Beispiel 1 beschrieben, aufgearbeitet.

```
Ausbeute:        197 - 201 g 5-Chlorcarbonyl-5H-dibenz/b,f/
                             azepin (II)
                 93 - 94,5% d.Theorie
```

Die Aufarbeitung der methanolischen Fällbadmutterlauge ergibt weitere 5 - 10 g Chlorcarbonyl-iminostilben (II).

Beispiel 6

160 g Iminostilben werden in 800 ml Toluen suspendiert. Bei ca. 40°C beginnt man mit dem Einleiten des Phosgens. Nach ca. 10-20%igem Umsatz des Iminostilbens erfolgt die langsame Zudosierung von verdünnter Natronlauge (24 g NaOH = 0,60 mol, gelöst in 200 ml Wasser). Die Geschwindigkeit der Phosgeneinleitung und die zudosierte Menge der verdünnten Natronlauge werden so aufeinander eingeregelt, daß der pH-Wert der Reaktionsmischung während der Laugezugabe nie in den alkalischen Bereich abgleitet. Die Phosgenierung wird bei 40-50°C durchgeführt.
Nach dem vollständigen Umsatz des Iminostilbens wird nach Beispiel 1 aufgearbeitet.

```
Ausbeute:  197 - 200 g 5-Chlorcarbonyl-5H-dibenz/b,f/azepin (II)
           93 -  94 % d. Theorie
F:         157 - 158°C
```

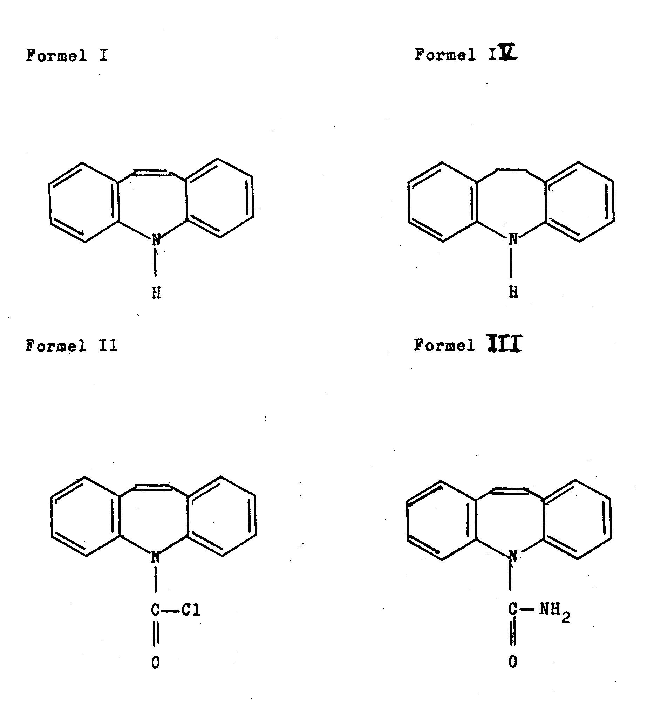

Patentansprüche

1.   Verfahren zur Herstellung von 5-Chlorcarbonyl-5H-dibenz/b,f/azepin durch Umsetzung von Iminostilben mit Phosgen in einem wasserfreien, aromatischen Lösungsmittel, dadurch gekennzeichnet, daß man Iminostilben in einem inerten Lösungsmittel bei Temperaturen von 20-60°C mit Phosgen umsetzt, aus dem neben dem 5-Chlorcarbonyl-5H-dibenz/b,f/azepin sich bildenden Iminostilbenhydrochlorid durch Zugabe geeigneter, wäßriger Basen Iminostilben freisetzt und dieses unter Beibehaltung saurer Reaktionsbedingungen vollständig phosgeniert.

2. Verfahren nach Anspruch 1, dadurch gekennzeichnet, daß man in die auf 20 -60°C, vorzugsweise auf 35 - 50°C, erwärmte Suspension von Iminostilben zunächst so viel Phosgen einleitet,bis ein nahezu äquimolekulares Gemisch aus 5-Chlorcarbonyl-5H-dibenz/b,f/azepin und Iminostilben-hydrochlorid entstanden ist.

3. Verfahren nach einem der Ansprüche 1 und 2, dadurch gekennzeichnet, daß als wäßrige Base Lösungen von Alkalihydroxiden, wäßrige Ammoniaklösung, wäßrige Lösungen von infolge Hydrolyse alkalisch reagierenden Salzen oder wäßrige Suspensionen von Erdalkalihydroxiden oder -oxiden verwendet werden.

4. Verfahren nach einem der Ansprüche 1 bis 3, dadurch gekennzeichnet, daß als inerte Lösungsmittel Aromaten oder Halogenaromaten, wie Toluen oder Chlorbenzen, verwendet werden.

5. Verfahren nach einem der Ansprüche 1 bis 4, dadurch gekennzeichnet, daß auf 1 Äquivalent Iminostilben 0,5 - 1,0 Äquivalente, vorzugsweise 0,55 - 0,75 Äquivalente Alkali bzw. Base eingesetzt werden.

Formel I

Formel IV

Formel II

Formel III

**Europäisches Patentamt**

**EUROPÄISCHER RECHERCHENBERICHT**

Nummer der Anmeldung

**EP 90 12 2448**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, Band 113, Nr. 1, 2. Juli 1990, Seite 600, Zusammenfassung Nr. 6185n, Columbus, Ohio, US; <br> & PL-A-147 078 (POLFA) 29-04-1989 <br> – – – – – | 1-5 | C 07 D 223/22 |
| | | | **RECHERCHIERTE SACHGEBIETE (Int. Cl.5)** |
| | | | C 07 D 223/00 |

Der vorliegende Recherchenbericht wurde für alle Patentansprüche erstellt

| Recherchenort | Abschlußdatum der Recherche | Prüfer |
|---|---|---|
| Den Haag | 26 September 91 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument
------------------------------------------------
& : Mitglied der gleichen Patentfamilie, übereinstimmendes Dokument